# FASCICULE DE BREVET EUROPEEN

(11) **EP 0 663 812 B1**
(45) Date de publication et mention de la délivrance du brevet: **02.10.1996**
(21) Numéro de dépôt: 92918038.8
(22) Date de dépôt: 05.08.1992
(51) Int. Cl.: A61K 7/06

(54) **UTILISATION D'ALKYLPOLYGLYCOSIDES ET/OU DE DERIVES O-ACYLES DU GLUCOSE POUR LE TRAITEMENT DE LA CHUTE DES CHEVEUX**
VERWENDUNG VON ALKYLPOLYGLYCOSIDEN UND/ODER 0-ACYLIERTE GLUKOSEDERIVATE ZUR BEHANDLUNG DES HAARAUSFALLS
USE OF ALKYLPOLYGLYCOSIDES AND/OR O-ACYLATED DERIVATIVES OF GLUCOSE FOR TREATING HAIR LOSS

(30) Priorité: 07.08.1991 FR 9110060
(43) Date de publication de la demande: 26.07.1995
(73) Titulaire: L'OREAL, F-75008 Paris (FR)
(72) Inventeur: DURANTON, Albert, F-75018 Paris (FR); HANSENNE, Isabelle, F-75017 Paris (FR)
(74) Mandataire: Casalonga, Axel
(86) Numéro de dépôt international: FR9200773
(87) Numéro de publication internationale: WO9302657

(56) Documents cités:
- EP-A- 0 357 484
- EP-A- 0 427 210
- EP-A- 0 428 157
- GB-A- 2 128 627

## Description

La présente invention a pour objet l'utilisation en cosmétique ou en pharmacie d'alkylpolyglycosides et/ou de dérivés O-acylés du glucose pour le traitement de la chute des cheveux.

La croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années au cours de laquelle les cheveux s'allongent, succède une phase catagène très courte et transitoire et une phase de repos appelée phase télogène de quelques mois.

A la fin de la période de repos, les cheveux tombent et un autre cycle recommence. La chevelure se renouvelle donc en permanence chez l'être humain et sur les 150.000 cheveux environ que comporte une chevelure, à chaque instant 10% environ sont au repos et seront donc remplacés en quelques mois.

Dans un nombre important de cas, la chute précoce des cheveux survient sur des sujets prédisposés génétiquement et elle atteint notamment les hommes. Il s'agit plus particulièrement de l'alopécie androgénétique ou androgénique ou encore androgéno-génétique.

Cette alopécie est essentiellement due à une perturbation du renouvellement capillaire qui entraîne, dans un premier temps, l'accélération de la fréquence des cycles aux dépens de la qualité des cheveux puis de leur quantité. Il y a un appauvrissement progressif de la chevelure par régression des cheveux dits "terminaux" au stade de duvets. Des zones sont touchées préférentiellement notamment les golfes temporaux ou frontaux chez l'homme, et chez les femmes, on constate une alopécie diffuse du vertex.

On recherche depuis de nombreuses années dans l'industrie cosmétique ou pharmaceutique, des compositions permettant de supprimer ou réduire l'effet de l'alopécie andro-génétique, et notamment d'induire ou de stimuler là croissance des cheveux ou de diminuer leur chute.

Dans cette optique, on a déjà proposé des composés tels que l'amino-6 dihydro- 1,2 hydroxy-1 imino-2 pipéridino-4 pyrimidine ou Minoxidil ainsi que ses dérivés, tels que décrits dans le brevet US-A-4.139.619.

On a également proposé des oligosaccharides contenant au moins une unité disaccharide constituée d'un reste d'acide uronique et d'un reste d'hexosamine. Ils sont décrits dans la demande de brevet européen EP O 211 61O.

La demanderesse vient de découvrir de manière surprenante que les composés alkylpolyglycosides et/ou les dérivés O-acylés du glucose permettaient d'induire et de stimuler la croissance des cheveux et de diminuer leur chute efficacement.

La demanderesse a par ailleurs constaté que les compositions contenant ces composés particuliers, présentaient une bonne stabilité au stockage et des propriétés cosmétiques améliorées, en particulier au niveau brillance et poudrage des cheveux et ne provoquaient pas d'irritation du cuir chevelu même après un contact prolongé, sans rinçage.

Un objet de l'invention consiste donc en l'utilisation en cosmétique d'alkylpolyglycosides et/ou de dérivés O-acylés du glucose pour le traitement de la chute des cheveux.

Un autre objet est constitué par l'utilisation de ces composés pour la préparation de compositions pharmaceutiques à base de ces composés particuliers pour le traitement de la chute des cheveux.

L'invention a également pour objet un procédé de traitement cosmétique des cheveux et du cuir chevelu grâce à ces composés.

D'autres objets apparaîtront à la lecture de la description et des exemples qui suivent.

L'utilisation conforme à l'invention met en oeuvre des composes répondant à la formule (I) :

R₁(C₆H₁₀O₅)ₓH (I)

correspondant à la structure développée (II) : 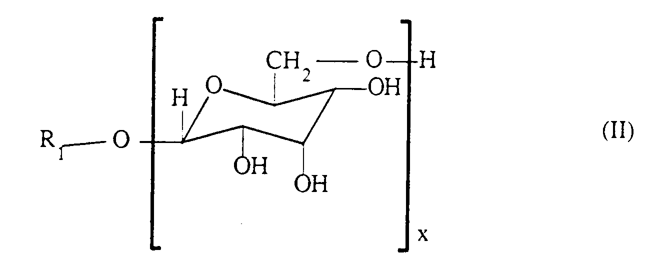 dans laquelle :
R₁ désigne un radical ou un mélange de radicaux alkyle ou alcényle à chaîne droite ou ramifiée en C₈-C₂₄;
x est un nombre compris entre 1 et 15;
et/ou des composés répondant à la formule (III) : 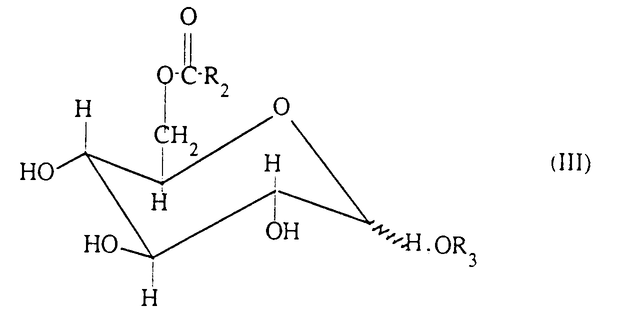 dans laquelle R₂ représente une chaîne hydrocarbonée, linéaire, saturée ou insaturée, contenant de 7 à 19 atomes de carbone et R₃ représente l'hydrogène ou un groupe alkyle inférieur en C₁-C₄; sous réserve que les composés de formule (I) ne soient pas utilisés en association avec des dérivés pyrimidiniques.

Les composés alkylpolyglycosides de formule (I) définie ci-dessus, utilisés conformément à l'invention, sont de préférence représentés par les produits vendus par la Société HENKEL sous la dénomination APG, tels que les produits APG 300, APG 350, APG 500, APG 550, APG 625, APG base 10-12; les produits vendus par la Société SEPPIC sous les dénominations TRITON CG 110 (ou ORAMIX CG 110) et TRITON CG 312 (ou ORAMIX NS 10); ceux vendus par la Société BASF sous la dénomination LUTENSOL GD 70.

Les composés de formule (III) sont des composés connus, dont un procédé de préparation est décrit ci-après, et ils sont choisis notamment dans le groupe de ceux dont le reste acyle R₂-CO- est un reste octanoyle, décanoyle, dodécanoyle, myristoyle, hexadécanoyle, stéaroyle, oléoyle, linoléoyle ou linolénoyle. A titre d'exemples de composés de formule (III), on peut mentionner le O-oléoyl-6-D-glucose. le O-décanoyl-6-D-glucose, le O-dodécanoyl-6-D-glucose et le O-hexadécanoyl-6-D-glucose.

Le mode opératoire général de la préparation de dérivés O-acylés en position 6 du D-glucose de formule (III) est le suivant :

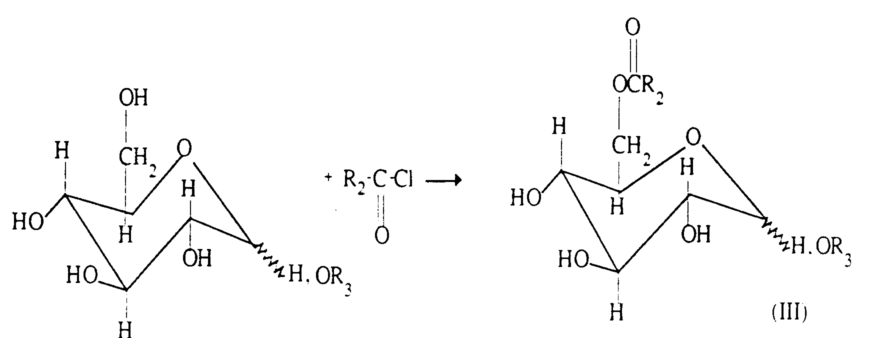

La synthèse est réalisée à partir du chlorure de l'acide choisi et du D-glucose selon la méthode décrite par E. REINEFELD & Coll., "Die Stärke". n°6, pages 181-189, 1968.

Les composés conformes à l'invention de formules (I) ou (III) peuvent être associés à des composés améliorant encore leur activité sur la repousse et/ou sur le freinage de la chute des cheveux, tels que plus particulièrement les composés suivants :
- les esters d'acide nicotinique, dont plus particulièrement les nicotinates d'alkyle en C₁-C₆ et notamment le nicotinate de méthyle, d'hexyle, de benzyle, etc..;
- les agents anti-inflammatoires stéroïdiens et non stéroïdiens bien connus dans l'état de la technique et en particulier l'hydrocortisone. ses sels et ses dérivés, l'acide niflumique, etc;
- les rétinoïdes comme l'acide t-trans rétinoïque appelé encore trétinoïne, l'isotrétinoïne, le rétinol ou vitamine A et ses dérivés, tels que l'acétate, le palmitate ou le propionate le motrétinide, l'étrétinate, le t-trans rétinoate de zinc;
- les dérivés de pyrimidine comme l'amino-6 dihydro-1,2 hydroxy-1 imino-2 pipéridino-4 pyrimidine encore connu sous le nom de "Minoxidil", tels que décrits dans le brevet US 4 139 619, présents uniquement pour les composés de formule (III);
- les agents antibactériens choisis parmi les macrolides, les pyranosides et les tétracyclines, et notamment l'érythromycine;
- les agents antagonistes de calcium, tels que la Cinnarizine et le Diltiazem;
- des hormones, telles que l'estriol ou des analogues ou la thyroxine et ses sels;
- des agents antiandrogènes tels que l'oxendolone, la spironolactone, le diéthylstilbestrol;
- des capteurs de radicaux OH, tels que le diméthylsulfoxyde.

On peut également associer avec les composés de l'invention, éventuellement en mélange avec les autres, des composés tels que le Diazoxyde correspondant au méthyl-3 chloro-7 [2H] benzothiadiazine-1,2,4 dioxyde-1,1; la Spiroxazone ou 7-(acétylthio)-4',5'-dihydrospiro [androst 4-ène-17,2'-(3'H)furan]-3 one; des phospliolipides, tels que la lécithine; les acides linoléique et linolénique; l'acide salicylique et ses dérivés décrits dans le brevet français 2 581 542, comme les dérivés d'acide salicylique porteurs d'un groupement alcanoyle ayant 2 à 12 atomes de carbone en position 5 du cycle benzénique; des acides hydroxycarboxyliques ou cétocarboxyliques et leurs esters; des lactones et leurs sels correspondants; l'anthraline; les caroténoïdes; les acides eicosatétraynoïque et eicosatriynoïque ou leurs esters et amides.

Les compositions conformes à l'invention contenant ces composés peuvent se présenter sous forme de lotions, d'émulsions, de crèmes, de gels, et éventuellement être pressurisées en aérosol. Elles peuvent être appliquées notamment dans les traitements mettant en oeuvre une composition telle que définie ci-dessus, dont l'application est ou non suivie d'un rinçage, ou encore sous forme de shampooing.

Dans les compositions non rincées, les composés de formule (I) ou les composés de formule (III) sont présents dans des proportions comprises entre 0,1 et 10% en poids par rapport au poids total de la composition et de préférence entre 0,25 et 5% en poids.

Dans les compositions suivies d'un rinçage, les mêmes composés sont présents dans des proportions comprises entre 1 et 30% en poids par rapport au poids total de la composition et de préférence entre 5 et 15% en poids.

Lorsque les compositions selon l'invention constituent un shampooing, elles peuvent contenir des agents tensio-actifs anioniques, non-ioniques, amphotères ou zwittérioniques.

Le milieu utilisé dans ces compositions peut être constitué par de l'eau ou un mélange d'eau et d'un solvant ou un mélange de solvants, les solvants étant choisis parmi les solvants organiques acceptables sur le plan cosmétique et plus particulièrement parmi les alcools inférieurs en C₁-C₄; les alkylèneglycols; les alkyléthers d'alkylèneglycol et de dialkylèneglycol. Les solvants, lorsqu'ils sont présents, le sont dans des proportions comprises entre 5 et 95% en poids par rapport au poids total de la composition.

Ces compositions peuvent contenir d'autres adjuvants habituellement utilisés dans le domaine cosmétique ou phamlaceutique, en vue de réaliser des compositions topiques, tels que des agents tensio-actifs, des agents épaississants, des agents cosmétiques tels, à titre d'exemple non limitatif, que des polymères, des protéines et plus particulièrement des huiles de synthèse des agents conservateurs, des agents alcalinisants ou acidifiants. Le pH de ces compositions peut varier entre 3 et 9 et de préférence entre 5 et 8.

Les agents épaississants ou gélifiants peuvent être choisis parmi les biopolysaccharides, tels que par exemple les gommes de xanthane et les scléroglucanes, les dérivés de cellulose tels que l'hydroxypropyl cellulose, la méthylcellulose, les acides polyacryliques réticulés ou non, les polyéthylèneglycols et leurs dérivés, les associations de polymères anioniques et de polymères cationiques, telles que celles décrites dans le brevet français n° 2 598 611.

Les épaississants sont présents de préférence dans des proportions comprises entre 0,1 et 5% en poids, et en particulier entre 0.4 et 3% en poids par rapport au poids total de la composition.

Les huiles de synthèse peuvent être choisies parmi les huiles suivantes ou leurs mélanges :
a) les isoparaffines répondant à la formule (1) : 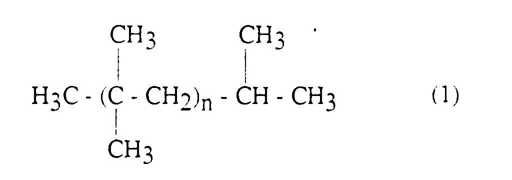 où n est compris entre 2 et 16 inclus;
b) le mélange des isoparaffines de formule (1) avec des isoparaffines de formule (2) : 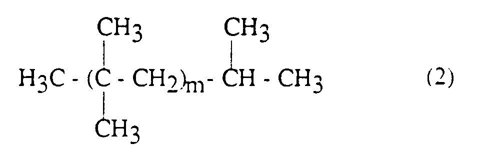 où m est égal ou supérieur à 18, et de préférence compris entre 18 et 40; la viscosité capillaire des huiles définies sous a) et b) étant inférieure à 500 cps.
c) Les polydécènes tels que par exemple les produits de viscosités à 40°C inférieures à 40 cst, vendus par la société ETHYL CORPORATION sous les dénominations "ETHYLFLO 362 NF, 364 NF, 366 NF".

Parmi les huiles synthétiques répondant à la formule (I) définie ci-dessus, on peut citer celles dans lesquelles n est égal à 2, 3, 4 ou 16, et en particulier les produits vendus sous les dénominations "PERMETHYL 99A, 101A, 102A ou 104A" par la Société PRESPERSE INC ou le produit ARLAMOL HD vendu par la Société ICI, correspondant à la formule (I) dans laquelle n est égal à 3.

Parmi les huiles synthétiques de formule (II), on peut citer le produit vendu sous la dénomination commerciale de "PERMETHYL 106A", répondant à la formule (II) dans laquelle m est égal à 38.

Les huiles de synthèse sont présentes dans des proportions comprises entre 0,05 et 20% et de préférence entre 0.1 et 10%.

Le procédé de traitement de la chute des cheveux consiste principalement à appliquer sur les zones alopéciques du cuir chevelu et des cheveux d'un individu, une composition telle que définie ci-dessus, à laisser en contact plusieurs heures et éventuellement à rincer.

On peut par exemple appliquer la composition sur les cheveux et le cuir chevelu, le soir, garder la composition toute la nuit au contact et éventuellement effectuer un shampooing le matin ou laver les cheveux à l'aide de cette composition et à laisser à nouveau au contact quelques minutes avant de rincer.

Le procédé de traitement présente les caractéristiques d'un procédé cosmétique dans la mesure où il permet de soigner les cheveux et le cuir chevelu au sens cosmétique du terme, c'est-à-dire leur apporter les substances qui leur manquent et les embellir.

Les compositions conformes à l'invention se sont révélées particulièrement intéressantes lorsqu'elles sont appliquées sous forme de lotions capillaires, éventuellement rincées ou de shampooings. Sous forme de shampooings, elles présentent un bon pouvoir moussant.

L'invention a également pour objet l'utilisation des composés de formule (I) ou des composés de formule (III) telles que définies ci-dessus pour la préparation d'un médicament destiné au traitement des maladies du cuir chevelu telles que l'alopécie, sous réserve que ledit médicament ne contient pas de dérivé pyrimidinique en présence de composés de formule (I) telle que définie ci-dessus .

L'invention a également pour object une composition telle que définie par les revendications 20-22. Les exemples suivants sont destinés à illustrer l'invention sans pour autant présenter un caractère limitatif.

### EXEMPLE 1

On prépare une lotion non rincée de composition suivante :

| | |
|---|---|
| - Alkyl(C₉-C₁₀-C₁₁/20-40-40)polyglycoside vendu à 50% de MA sous la dénomination APG 300 par la Société HENKEL | 3 g MA |
| - Alcool éthylique | 30.55 g |
| - Parfum qs | |
| - Eau qsp | 100 g |

### EXEMPLE 2

On prépare un shampooing de composition suivante :

| | |
|---|---|
| - Alkyl(C₁₂-C₁₄-C₁₆/68-26-6)polyglycoside vendu à 50% de MA sous la dénomination APG 600 par la Société HENKEL | 20 g MA |
| - Hydroxypropylcellulose (PM 300.000) vendue sous la dénomination KLUCEL G par la Société AQUALON | 0.5 g |
| - Alcool éthylique | 50 g |
| - Conservateurs, parfum qs | |
| - Eau qsp | 100 g |

### EXEMPLE 3

On prépare une lotion non rincée de composition suivante :

| | |
|---|---|
| - Alkyl(C₉-C₁₀-C₁₁/20-40-40)polyglycoside vendu à 50% de MA sous la dénomination APG 300 par la Société HENKEL | 5 g MA |
| - Propylèneglycol | 22.8 g |
| - Alcool éthylique | 55.1 g |
| - Eau qsp | 100 g |

### EXEMPLE 4

On prépare une lotion non rincée de composition suivante :

| | |
|---|---|
| - O-octanoyl-6D-glucose | 5 g |
| - Propylèneglycol | 22.8 g |
| - Alcool éthylique | 55.1 g |
| - Eau qsp | 100 g |

### EXEMPLE 5

On prépare une lotion non rincée de composition suivante :

| | |
|---|---|
| - O-oléoyl-6D-glucose | 2,5 g |
| - Propylèneglycol | 22,8 g |
| - Alcool éthylique | 55.1 g |
| - Eau qsp | 100 g |

### EXEMPLE 6

On prépare une lotion non rincée de composition suivante :

| | |
|---|---|
| - Alkyl(C₁₂-C₁₃)polyglycoside vendu à 50% de MA sous la dénomination APG 500 par la Société HENKEL | 5 g MA |
| - Propylèneglycol | 22,8 g |
| - Alcool éthylique | 55,1 g |
| - Eau qsp | 100 g |

### EXEMPLE 7

On prépare une lotion non rincée de composition suivante :

| | |
|---|---|
| - O-octanoyl-6-D-glucose | 2,5 g |
| - Alkyl(C₁₂-C₁₃)glucoside vendu à 50% de MA sous la dénomination APG 500 par la Société HENKEL | 2,5 g MA |
| - Propylèneglycol | 22,8 g |
| - Alcool éthylique | 55,1 g |
| - Eau qsp | 100 g |

### EXEMPLE 8

On prépare un shampooing antichute de composition suivante :

| | |
|---|---|
| - Alkyl(C₉-C₁₀-C₁₁/20-40-40)polyglycoside vendu à 50% de MA sous la dénomination APG 300 par la Société HENKEL | 15 g MA |
| - Gomme de xanthane vendu sous la dénomination KELTROL T par la société RHONE POULENC | 1 g |
| - Huile de synthèse vendue par la société ICI sous la dénomination ARLAMOL HD | 2 g |
| - Conservateur, parfum qs | |
| - HCl qs pH : 7 | |
| - Eau qsp | 100 g |

## Revendications

1. Utilisation pour le traitement cosmétique de la chute des cheveux, de composés de formule :
R₁(C₆H₁₀O₅)ₓ H (I)
correspondant à la structure développée (II) : 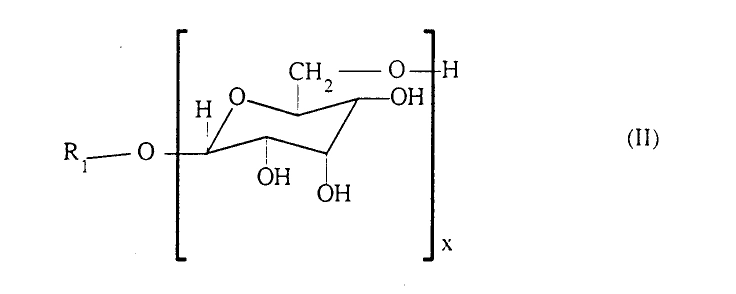 dans laquelle :
R₁ désigne un radical ou un mélange de radicaux alkyle ou alcényle à chaîne droite ou ramifiée en C₈-C₂₄,
x est un nombre compris entre 1 et 15;
et/ou de composés répondant à la formule (III) : 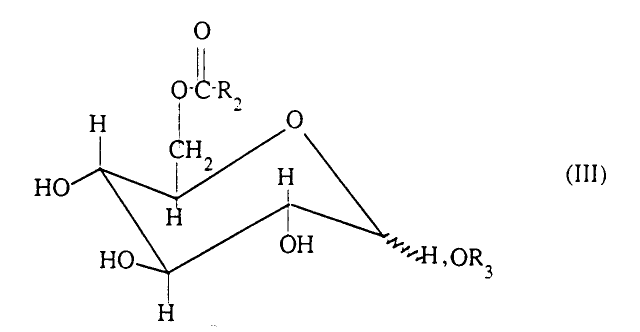 dans laquelle R₂ représente une chaîne hydrocarbonée, linéaire, saturée ou insaturée, contenant de 7 à 19 atomes de carbone et R₃ représente l'hydrogène ou un groupement alkyle inférieur en C₁ à C₄; sous réserve que les composés de formule (I) ne soient pas utilisés en présence de dérivés de pyrimidine.

2. Utilisation selon la revendication 1, caractérisée par le fait que les composés de formule (III) sont choisis parmi ceux pour lesquels le radical acyle R₂-CO- désigne un radical octanoyle, décanoyle, dodécanoyle, myristoyle, hexadecanoyle, stéaroyle, oléoyle, linoléoyle ou linolénoyle.

3. Composition cosmétique pour le traitement de la chute des cheveux, caractérisée par le fait qu'elle contient dans un milieu cosmétiquement acceptable, au moins un composé de formule (I) telle que définie dans la revendication 1, sous réserve qu'elle ne contienne pas de dérivé de pyrimidine.

4. Composition selon la revendication 3, caractérisée par le fait quelle contient en plus des agents améliorant l'activité sur la repousse des cheveux et/ou sur le freinage de leur chute.

5. Composition cosmétique pour le traitement de la chute des cheveux, caractérisée par le fait qu'elle contient dans un milieu cosmétiquement acceptable, au moins un composé de formule (I) et un composé de formule (III) tels que définis dans la revendication 1.

6. Composition cosmétique pour le traitement de la chute des cheveux, caractérisée par le fait qu'elle contient dans un milieu cosmétiquement acceptable, au moins un compose de formule (III) telle que définie dans la revendication 1 ou 2, et des agents améliorant l'activité sur la repousse des cheveux et/ou sur le freinage de leur chute.

7. Composition selon la revendication 4 ou 6, caractérisée par le fait que les agents améliorant l'activité sur la repousse des cheveux sont choisis parmi les esters d'acide nicotinique; les agents anti-inflammatoires stéroïdiens ou non; les rétinoïdes; les agents anti-bactériens; les antagonistes du calcium; les hormones; les agents antiandrogènes; les capteurs des radicaux -OH; les dérivés de pyrimidine lorsque la composition contient des composés de formule (III).

8. Composition selon l'une quelconque des revendications 3 à 7, caractérisée par le fait qu'elle contient en plus le méthyl-3 chloro-7 [2H]benzothiadiazine-1,2,4 dioxyde-1,1; la 7-(acétylthio)-4',5'-dihydrospiro[androst 4-ène-17,2'-(3'H)furan]-3 one; des phospholipides, tels que la lécithine: les acides linoléique et linolénique; l'acide salicylique et ses dérivés, et plus particulièrement les dérivés d'acide salicylique porteurs d'un groupement alcanoyle ayant 2 à 12 atomes de carbone en position 5 du cycle benzénique; des acides hydroxycarboxyliques ou cétocarboxyliques et leurs esters; des lactones et leurs sels correspondants; l'anthraline; les caroténoïdes; les acides eicosatétraynoïque et eicosatriynoïque ou leurs esters et amides.

9. Composition selon l'une quelconque des revendications 3 à 8, caractérisée par le fait qu'elle se présente sous forme de lotion, d'émulsion, de crème, de gel, éventuellement pressurisée dans un dispositif aérosol.

10. Composition selon l'une quelconque des revendications 3 à 9, dont l'application n'est pas suivie d'un rinçage, caractérisée par le fait qu'elle contient le composé de formule (I) et/ou le composé de formule (III) dans des proportions comprises entre 0.1 et 10% en poids par rapport au poids total de la composition.

11. Composition selon l'une quelconque des revendications 3 à 10, dont l'application est suivie d'un rinçage, caractérisée par le fait qu'elle contient le composé de formule (I) et/ou le composé de formule (III) dans des proportions comprises entre 1 et 30% en poids par rapport au poids total de la composition.

12. Composition selon l'une quelconque des revendications 3 à 11, caractérisée par le fait que le milieu cosmétiquement acceptable est constitué par de l'eau, un mélange d'eau et d'un solvant organique cosmétiquement acceptable.

13. Composition selon la revendication 12, caractérisée par le fait qu'elle contient un solvant dans des proportions comprises entre 5 et 95% en poids par rapport au poids total de la composition.

14. Composition selon l'une quelconque des revendications 3 à 13, caractérisée par le fait qu'elle contient des agents tensio-actifs anioniques, non-ioniques, amphotères ou zwittérioniques; des agents épaississants, des polymères, des protéines, des huiles de synthèse, des agents conservateurs, des agents alcalinisants ou acidifiants ou d'autres adjuvants habituellement utilisés en cosmétique ou en pharmacie.

15. Composition selon la revendication 14, caractérisée par le fait qu'elle contient des agents épaississants dans des proportions comprises entre 0,1 et 5% en poids par rapport au poids total de la composition.

16. Composition selon la revendication 14, caractérisée par le fait qu'elle contient une ou un mélange d'huile(s) de synthèse dans des proportions comprises entre 0.05 et 20% en poids par rapport au poids total de la composition.

17. Composition selon la revendication 16, caractérisée par le fait que l'huile de synthèse est choisie parmi
(a) les isoparaffines de formule : 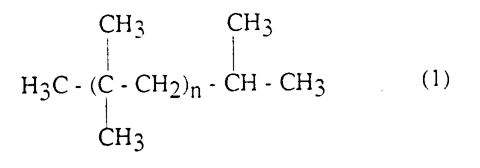 où n est compris entre 2 et 16 inclus;
(b) les mélanges d'isoparaffines de formule (1) avec des isoparaffines de formule : 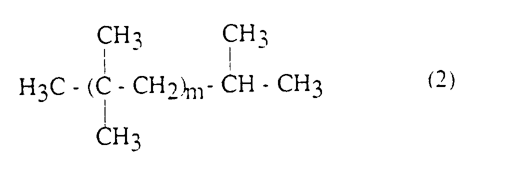 où m est égal ou supérieur à 18;
(c) les polydécènes; ou leurs mélanges.

18. Procédé de traitement cosmétique des cheveux, caractérisé par le fait que l'on applique sur les cheveux ou le cuir chevelu, au moins une composition selon l'une quelconque des revendications 3 à 14, qu'on la laisse en contact avec les cheveux ou le cuir chevelu, et qu'éventuellement, on rince.

19. Utilisation des composés de formule (I) et/ou des composés de formule (III) définis dans la revendication 1 ou 2, pour la préparation d'un médicament destine' au traitement de la chute des cheveux; sous reserve que ledit médicament ne contienne pas de dérivé de pyrimidine en présence des composés de formule (I).

20. Composition pour le traitement thérapeutique par voie topique de la chute des cheveux, caractérisée par le fait qu'elle comprend dans un milieu pharmaceutiquement acceptable, au moins un composé de formule (I) tel que défini dans la revendication 1, sous réserve qu'elle ne contient pas de dérivé pyrimidinique.

21. Composition pour le traitement thérapeutique par voie topique de la chute des cheveux, caractérisée par le fait qu'elle contient dans un milieu pharmaceutiquement acceptable, au moins un composé de formule (III) tel que défini dans la revendication 1 ou 2, et des agents améliorant l'activité sur la repousse des cheveux et/ou sur le freinage de leur chute.

22. Composition pour le traitement thérapeutique par voie topique de la chute des cheveux, caractérisée par le fait qu'elle contient au moins un composé de formule (I) et un composé de formule (III) tels que définis dans la revendication 1.

## Claims

1. Use for the cosmetic treatment of hair loss of compounds of formula:
R₁(C₆H₁₀O₅)ₓH (I)
corresponding to the structural formula (II): 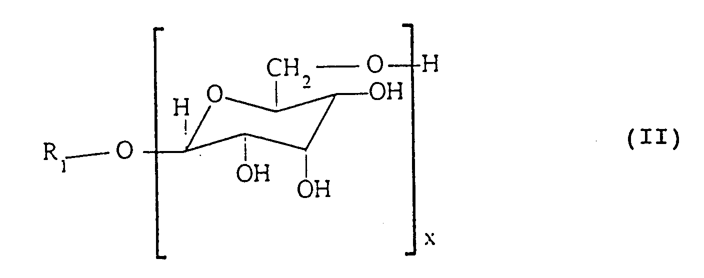 in which:
R₁ denotes an alkyl or alkenyl radical having an unbranched or branched C₈-C₂₄ chain, or a mixture of such radicals;
x is a number between 1 and 15;
and/or of compounds corresponding to the formula (III): 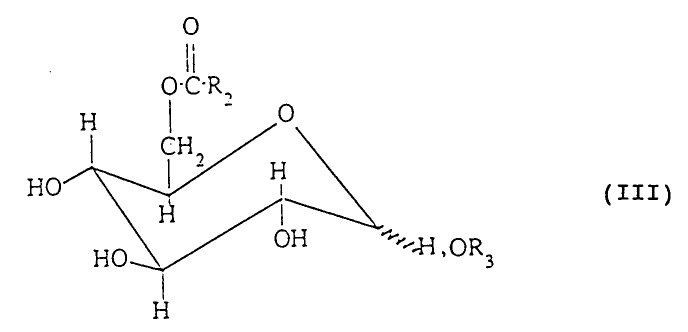 in which R₂ represents a saturated or unsaturated, linear hydrocarbon chain containing from 7 to 19 carbon atoms and R₃ represents hydrogen or a C₁ to C₄ lower alkyl group; with the proviso that the compounds of formula (I) are not used in the presence of pyrimidine derivatives.

2. Use according to claim 1, characterized in that the compounds of formula (III) are chosen from those for which the acyl radical R₂-CO- denotes an octanoyl, decanoyl, dodecanoyl, myristoyl, hexadecanoyl, stearoyl, oleoyl, linoleoyl or linolenoyl radical.

3. Cosmetic composition for treating hair loss, characterized in that it contains at least one compound of formula (I) as defined in claim 1 in a cosmetically acceptable medium, with the proviso that it does not contain a pyrimidine derivative.

4. Composition according to claim 3, characterized in that it contains, in addition, agents that improve the activity with respect to hair regrowth and/or to the halting of hair loss.

5. Cosmetic composition for treating hair loss, characterized in that it contains at least one compound of formula (I) and one compound of formula (III) as are defined in claim 1 in a cosmetically acceptable medium.

6. Cosmetic composition for treating hair loss, characterized in that it contains at least one compound of formula (III), as defined in claim 1 or 2, and agents that improve the activity with respect to hair regrowth and/or to the halting of hair loss, in a cosmetically acceptable medium.

7. Composition according to claim 4 or 6, characterized in that the agents that improve the activity with respect to hair regrowth are chosen from nicotinic acid esters, steroidal or nonsteroidal anti-inflammatory agents; retinoids; antibacterial agents; calcium antagonists; hormones; antiandrogens; -OH radical trapping agents; and pyrimidine derivatives when the composition contains compounds of the formula (III).

8. Composition according to any one of claims 3 to 7, characterized in that it contains, in addition, 3-methyl-7-chloro-[2H]-1,2,4-benzothiadiazine 1,1-dioxide; 7-(acetylthio)-4'5'-dihydrospiro[4-androstene-17,2'(3H)-furan]-3-one; phospholipids such as lecithin; linoleic and linolenic acids; salicylic acid and its derivatives, and more especially salicylic acid derivatives bearing an alkanoyl group having 2 to 12 carbon atoms at position 5 of the benzene ring; hydroxycarboxylic and ketocarboxylic acids and their esters; lactones and their corresponding salts; anthralin; carotenoids; and icosatetraynoic and icosatriynoic acids or their esters and amides.

9. Composition according to any one of claims 3 to 8, characterized in that it takes the form of a lotion, emulsion, cream or gel, pressurized where appropriate in an aerosol device.

10. Composition according to any one of claims 3 to 9, the application of which is not followed by a rinse, characterized in that it contains the compound of formula (I) and/or the compound of formula (III) in proportions of between 0.1 and 10% by weight relative to the total weight of the composition.

11. Composition according to any one of claims 3 to 10, the application of which is followed by a rinse, characterized in that it contains the compound of formula (I) and/or the compound of formula (III) in proportions of between 1 and 30% by weight relative to the total weight of the composition.

12. Composition according to any one of claims 3 to 11, characterized in that the cosmetically acceptable medium consists of water or a mixture of water and a cosmetically acceptable organic solvent.

13. Composition according to claim 12, characterized in that it contains a solvent in proportions of between 5 and 95% by weight relative to the total weight of the composition.

14. Composition according to any one of claims 3 to 13, characterized in that it contains anionic, nonionic, amphoteric or zwitterionic surfactants; thickening agents, polymers, proteins, synthetic oils, preservatives, alkalinizing or acidifying agents or other adjuvants customarily used in cosmetics or in pharmacy.

15. Composition according to claim 14, characterized in that it contains thickening agents in proportions of between 0.1 and 5 % by weight relative to the total weight of the composition.

16. Composition according to claim 14, characterized in that it contains a synthetic oil or a mixture of synthetic oils in proportions of between 0.05 and 20% by weight relative to the total weight of the composition.

17. Composition according to claim 16, characterized in that the synthetic oil is chosen from
(a) the isoparaffins of formula: 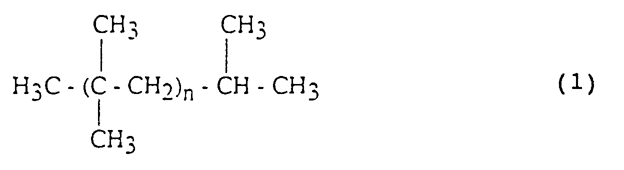 where n is between 2 and 16 inclusive;
(b) mixtures of isoparaffins of formula (1) with isoparaffins of formula: 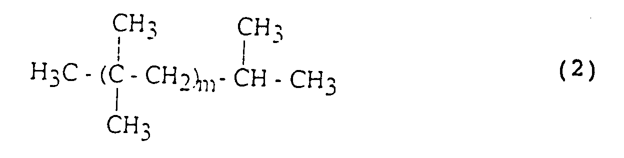 where m is equal to or greater than 18;
(c) polydecenes; or mixtures thereof.

18. Process for cosmetic treatment of the hair, characterized in that at least one composition according to any one of claims 3 to 14 is applied to the hair or scalp, in that it is left in contact with the hair or scalp and in that, if appropriate, the head is rinsed.

19. Use of the compounds of formula (I) and/or of the compounds of formula (III) which are defined in claim 1 or 2, for the preparation of a medicinal product intended for the treatment of hair loss; with the proviso that said medicinal product does not contain a pyrimidine derivative in the presence of the compounds of formula (I).

20. Composition for the topical therapeutic treatment of hair loss, characterized in that it comprises at least one compound of formula (I) as defined in claim 1 in a pharmaceutically acceptable medium, with the proviso that it does not contain a pyrimidine derivative.

21. Composition for the topical therapeutic treatment of hair loss, characterized in that it contains at least one compound of formula (III) as defined in claim 1 or 2, and agents that improve the activity with respect to hair regrowth and/or to the halting of hair loss, in a pharmaceutically acceptable medium.

22. Composition for the topical therapeutic treatment of hair loss, characterized in that it contains at least one compound of formula (I) and one compound of formula (III) as are defined in claim 1.

## Patentansprüche

1. Zur kosmetischen Behandlung des Ausfalls von Haaren vorgesehene Verwendung von Verbindungen der Formel :
R₁(C₆H₁₀O₅)ₓH (I)
entsprechend der dargestellten Struktur (II): 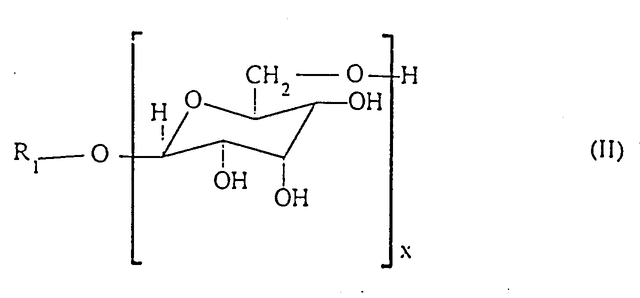 worin gilt:
R₁ bedeutet einen Alkyl- oder Alkenylrest mit gerader oder verzweigter C₈₋₂₄-Kette oder eine Mischung dieser Reste
x ist eine Zahl von 1 bis 15,
und/oder von Verbindungen der Formel (III): 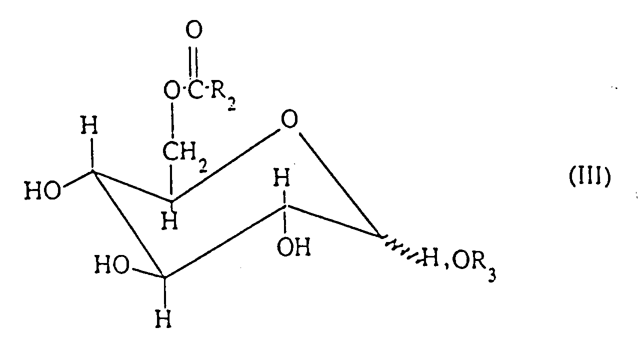 worin R₂ eine lineare, gesättigte oder ungesättigte Kohlenwasserstoffkette mit 7 bis 19 Kohlenstoffatomen und R₃ Wasserstoff oder eine C₁₋₄-Niedrigalkylgruppe darstellen, mit der Maßgabe, daß die Verbindungen der Formel (I) nicht zusammen mit Pyrimidin-Derivaten verwendet werden.

2. Verwendung gemäß Anspruch 1,
dadurch **gekennzeichnet**, daß
die Verbindungen der Formel (III) aus denjenigen ausgewählt sind, für die der Acylrest R₂-CO- einen Octanoyl-, Decanoyl-, Dodecanoyl-, Myristoyl-, Hexadecanoyl-, Stearoyl-, Oleoyl-, Linoleoyl- oder Linolenoyl-Rest bedeutet.

3. Kosmetische Zusammensetzung zur Behandlung des Ausfalls von Haaren,
dadurch **gekennzeichnet**, daß
sie in einem kosmetisch geeigneten Milieu mindestens eine Verbindung der in Anspruch 1 definierten Formel (I) enthält, mit der Maßgabe, daß sie kein Pyrimidin-Derivat enthält.

4. Zusammensetzung gemäß Anspruch 3,
dadurch **gekennzeichnet**, daß
sie zusätzlich Mittel enthält, die die Wirksamkeit bezüglich eines erneuten Wachstums der Haare und/oder der Verminderung von deren Ausfall verbessern.

5. Kosmetische Zusammensetzung zur Behandlung des Ausfalls der Haare,
dadurch **gekennzeichnet**, daß
sie in einem kosmetisch geeigneten Milieu mindestens eine Verbindung der Formel (I) und eine Verbindung der Formel (III), welche in Anspruch 1 definiert sind, enthält.

6. Kosmetische Zusammensetzung zur Behandlung des Ausfalls der Haare,
dadurch **gekennzeichnet**, daß
sie in einem kosmetisch geeigneten Milieu mindestens eine Verbindung der in Anspruch 1 oder 2 definierten Formel (III) sowie Mittel enthält, die die Wirksamkeit bezüglich eines erneuten Wachstums der Haare und/oder der Verminderung von deren Ausfall verbessern.

7. Zusammensetzung gemäß Anspruch 4 oder 6,
dadurch **gekennzeichnet**, daß
die Mittel, die die Wirksamkeit bezüglich des erneuten Wachstums der Haare verbessern, aus Nikotinsäureestern, gegebenenfalls steroiden entzündungshemmenden Mitteln, Retinoiden, antibakteriellen Mitteln, Calcium-Antagonisten, Hormonen, antiandrogenen Mitteln, Abfangmitteln für -OH-Radikale oder auch, wenn die Zusammensetzung Verbindungen der Formel (III) enthält, aus Pyrimidin-Derivaten ausgewählt sind.

8. Zusammensetzung gemäß einem der Ansprüche 3 bis 7,
dadurch **gekennzeichnet**, daß
sie zusätzlich 3-Methyl-7-Chlor-(2H)benzthiadiazin-1,2,4-dioxid-1,1, 7-Acetylthio-4',5'-dihydrospiro(androst-4-en-17,2'-(3'H)furan)-3-on, Phospholipide wie Lecithin, Linol- und Linolensäuren, Salicylsäure und ihre Derivate, insbesondere Salicylsäurederivate mit einer Alkanoylgruppe mit 2 bis 12 Kohlenstoffatomen in Position 5 des Benzolrings, Hydroxycarboxyl- oder Ketocarboxylsäuren und deren Estern, Lactone und deren entsprechende Salze, Anthralin, Karotinoide, Eicosatetrain- und Eicosatriinsäuren oder deren Ester und Amide enthält.

9. Zusammensetzung gemäß einem der Ansprüche 3 bis 8,
dadurch **gekennzeichnet**, daß
sie in Form einer Lotion, Emulsion, Creme, eines Gels oder gegebenenfalls unter Druck gesetzt in einer Aerosol-Vorrichtung vorliegt.

10. Zusammensetzung gemäß einem der Ansprüche 3 bis 9, die nicht zur Spülung vorgesehen ist,
dadurch **gekennzeichnet**, daß
sie die Verbindung der Formel (I) und/oder die Verbindung der Formel (III) in Mengenanteilen von 0,1 bis 10 Gew.% enthält, bezogen auf das Gesamtgewicht der Zusammensetzung.

11. Zusammensetzung gemäß einem der Ansprüche 3 bis 9, die zur Spülung vorgesehen ist,
dadurch **gekennzeichnet**, daß
sie die Verbindung der Formel (I) und/oder die Verbindung der Formel (III) in Mengenanteilen von 1 bis 30 Gew.% enthält, bezogen auf das Gesamtgewicht der Zusammensetzung.

12. Zusammensetzung gemäß einem der Ansprüche 3 bis 11,
dadurch **gekennzeichnet**, daß
das kosmetisch geeignete Milieu aus Wasser oder aus einer Mischung aus Wasser und einem kosmetisch geeigneten Lösungsmittel zusammengesetzt ist.

13. Zusammensetzung gemäß Anspruch 12,
dadurch **gekennzeichnet**, daß
sie ein Lösungsmittel in Mengenanteilen von 5 bis 95 Gew.% enthält, bezogen auf das Gesamtgewicht der Zusammensetzung.

14. Zusammensetzung gemäß einem der Ansprüche 3 bis 13,
dadurch **gekennzeichnet**, daß
sie anionische, nicht-ionische, amphotere oder zwitterionische oberflächenaktive Mittel, Verdickungsmittel, Polymere, Proteine, Syntheseöle, Konservierungsmittel, alkalisch oder sauer stellende Mittel oder weitere gewöhnlich in der Kosmetik oder Pharmazie verwendete Hilfsstoffe enthält.

15. Zusammensetzung gemäß Anspruch 14,
dadurch **gekennzeichnet**, daß
sie Verdickungsmittel in Mengenanteilen von 0,1 bis 5 Gew.% enthält, bezogen auf das Gesamtgewicht der Zusammensetzung.

16. Zusammensetzung gemäß Anspruch 14,
dadurch **gekennzeichnet**, daß
sie ein Syntheseöl oder eine Mischung aus Syntheseölen in Mengenanteilen von 0,05 bis 20 Gew.% enthält, bezogen auf das Gesamtgewicht der Zusammensetzung.

17. Zusammensetzung gemäß Anspruch 16,
dadurch **gekennzeichnet**, daß
das Syntheseöl ausgewählt ist aus:
(a) Isoparaffinen der Formel: 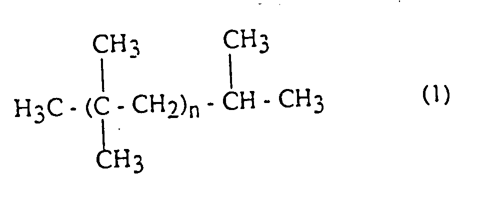 worin n 2 bis 16 beträgt,
(b) Mischungen aus Isoparaffinen der Formel (1) mit Isoparaffinen der Formel: 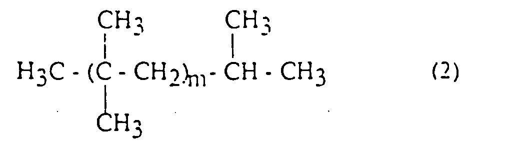 worin m gleich 18 oder mehr ist,
(c) Polydecenen, oder aus deren Mischungen.

18. Verfahren zur kosmetischen Behandlung der Haare,
dadurch **gekennzeichnet**, daß
man auf die Haare oder die behaarte Haut mindestens eine Zusammensetzung gemäß einem der Ansprüche 3 bis 14 aufbringt, diese in Kontakt mit den Haaren oder der behaarten Haut beläßt und gegebenenfalls spült.

19. Verwendung von Verbindungen der Formel (I) und/oder von Verbindungen der Formel (III), welche in Anspruch 1 oder 2 definiert sind, zur Herstellung eines Medikaments zur Behandlung des Haarausfalls, mit der Maßgabe, daß das genannte Medikament kein Pyrimidin-Derivat zusammen mit bzw. in Gegenwart von Verbindungen der Formel (I) enthält.

20. Zusammensetzung zur therapeutischen Behandlung des Ausfalls von Haaren auf topischem Weg,
dadurch **gekennzeichnet**, daß
sie in einem pharmazeutisch geeigneten Milieu mindestens eine Verbindung der in Anspruch 1 definierten Formel (I) enthält, mit der Maßgabe, daß sie kein Pyrimidin-Derivat enthält.

21. Zusammensetzung zur therapeutischen Behandlung des Haarausfalls auf topischem Wege,
dadurch **gekennzeichnet**, daß
sie in einem pharmazeutisch geeigneten Milieu mindestens eine Verbindung der in Anspruch 1 oder 2 definierten Formel (III) sowie Mittel enthält, die die Wirksamkeit bezüglich des erneuten Wachstums der Haare und/oder der Verminderung von deren Ausfall verbessern.

22. Zusammensetzung zur therapeutischen Behandlung des Ausfalls der Haare auf topischem Wege,
dadurch **gekennzeichnet**, daß
sie mindestens eine Verbindung der Formel (I) und eine Verbindung der Formel (III) enthält, wie sie in Anspruch 1 definiert sind.
